# EUROPEAN PATENT SPECIFICATION

(11) **EP 1 467 761 B1**
(45) Date of publication and mention of the grant of the patent: **02.11.2006**
(21) Application number: 02787238.1
(22) Date of filing: 29.11.2002
(51) Int. Cl.: A61K 45/00, A61P 27/00

(54) **TREATMENT OF NEOVASCULAR OPHTHALMIC DISEASE**
BEHANDLUNG VON OKULAREN NEOVASKULAREN ERKRANKUNGEN
TRAITEMENT DE MALADIES OPHTALMIQUES NEOVASCULAIRES

(30) Priority: 30.11.2001 US 335287 P
(43) Date of publication of application: 20.10.2004
(73) Proprietor: QLT, Inc., Vancouver, British Columbia V5T 4T5 (CA)
(72) Inventor: MARGARON, Philippe, Maria, Clotaire, Vancouver, British Columbia V6P 3M9 (CA); TAO, Jing-Song, Vancouver, British Columbia V6L 1B1 (CA)
(74) Representative: Brasnett, Adrian Hugh
(86) International application number: PCT/CA2002/001859
(87) International publication number: WO 2003/045432

(56) References cited:
- WO-A-01/83481
- US-B1- 6 214 813
- OIKAWA T ET AL: "POTENT INHIBITION OF ANGIOGENESIS BY WORTMANNIN, A FUNGAL METABOLITE" EUROPEAN JOURNAL OF PHARMACOLOGY, AMSTERDAM, NL, vol. 318, 1996, pages 93-96, XP002906145 ISSN: 0014-2999

## Description

### TECHNICAL FIELD

The invention relates to the use of inhibitors of integrin-linked kinase (ILK) in the treatment of various eye diseases with underlining pathology of neovascularization of cornea, iris, retina or choroid.

### BACKGROUND OF THE INVENTION

Vision is fundamentally important throughout life. However, the eye can be a fragile organ, and is susceptible to a number of hereditary and/or age related degenerative disorders. In the United States, a common cause of irreversible blindness or severe loss of vision is retinal dystrophies. The retina is the sensory tunic of the eye, containing light sensitive receptors, a complex of neurons, and pigmented epithelium, arranged in discrete layers. In humans, the macula is the portion of the retina that lies directly behind the lens. Cones, the photoreceptor cells responsible for central vision, are heavily concentrated in the macula. The peripheral retina is composed mainly of rods, which are responsible for side and night vision.

Neovascularization occurs in many eye diseases. Due to its significant epidemiology impact especially among aging population, it is becoming an important public health issue. Neovascularization, a pathological change characterized by an uncontrolled growth of vascular tissue, could occur in various parts of eye, including the cornea, iris, retina, and choroid. The consequences of neovascularization within these delicate ocular tissues are fibrosis, exudation, and/or hemorrhage that are responsible for vision loss in many common eye diseases.

Corneal neovascularization is characterized by invasion of vascular capillaries from the limbal vascular plexus into normally avascular cornea. In some cases, corneal neovascularization is associated with a decrease in visual acuity. The etiology of the corneal neovascularization is not fully understood. However, it is generally considered as a consequence of mechanical or chemical injury, or secondary to infection. Corneal neovascularization is associated with a variety of clinical conditions including contact lens wear, trauma and prior surgery (e.g., corneal transplant), viral, bacterial or protozoa infections, alkali burns and some immunologic diseases.

Iris neovascularization is characterized by the formation of leaky new blood vessels on the anterior surface of the iris and in the chamber angel recess. In the late stage of the disease, the vessels are enlarged and are accompanied by fibrous tissue, hence occluding the angle and causing the secondary neovascular glaucoma, a condition characterized by high intraocluar pressure, neovascularization of the iris and trabacular meshwork. Iris neovascularization and consequent neovascular glaucoma respond poorly to therapies and are frequent causes of blindness and enucleation. Iris neovascularization is associated with a variety of systemic and ocular diseases and secondary to trauma or therapies including surgery and radiation. Central retinal vein occlusion and diabetes mellitus are considered as leading causes of iris neovascularization.

Neovascularization of the retina involves the growth of new capillaries from the vessels that arise from the optic disk or inner retina. In the later stage, vision loss may occur due the development of various complications including scarring, tractional detachment of the retina, and hemorrhage.

Retinal neovascularization is associated with a variety of ocular and systemic diseases. Among those, diabetes mellitus, retinopathy of prematurity, central retinal vein occlusion, branch retinal vein occlusion and sickle cell disease are most frequently associated with retinal neovascularization.

Choroidal neovascularization (CNV) is characterized by an invasion of new blood vessels through Bruch's membrane. The consequence of CNV is severe and irreversible vision loss. CNV is associated with a variety of ocular diseases including degenerative conditions, inflammatory or infectious diseases and trauma. Age-related macular degeneration (AMD), angioid streaks, pathological myopia, ocular histoplasmosis syndrome, sarcoidosis and chronic uveitis are just a few examples of ocular conditions with choroidal neovascularization as a significant underlining pathological change.

The current treatment for many forms of ocular neovascularization involves photocoagulation or cryotherapy. Pan-retinal or focal photocoagulation is current standard therapy for diabetic retinopathy. It is partially effective in reducing the rate of vision loss in patients with diabetic retinopathy. Photocoagulation is also a destructive treatment with unwanted side effects, such as CNV, subretinal fibrosis, photocoagulation scar expansion, and inadvertent foveolar burns, that can cause loss of central visual acuity and scotoma formation. Patients with good visual acuity are less likely to recognize the benefits from this aggressive treatment and more likely to notice its side effects, which can include some loss of central and peripheral vision, and a reduction in color and night vision..

Visudyne^{™} therapy, an ocular application of photodynamic therapy (PDT) using photosensitizer verteporfin (Visudyne^{™}, Novartis Ophthalmics) and red light (690 nm) is a treatment of choice for patients with predominantly classic lesions of the wet form of AMD. It is also effective in treating occult subfoveal choroidal neovascularization secondary to AMD. However, in many patients the treatment needs to be repeated at three-month intervals over the subsequent one to two years, due to recurring CNV. The causes of the recurring vascular growth are not fully understood and may involve multiple factors including the induction of growth factors or inflammatory mediators following PDT.

Wortmannin is described by Oikawa et al., Eur. J. Pharmacol. **318**:93-96 (1996) as being a potent and selective inhibitor of PI3K with potential uses in the treatment of angiogenesis-dependent disorders. Likewise, WO 01/83481 describes the use of a novel class of PI3K inhibitors that may be used to inhibit abnormal cell growth in which PI3K plays a role.

The further development of treatments for neovascular ophthalmic disease is of great interest.

### SUMMARY OF THE INVENTION

The present invention provides the use of an inhibitor of integrin linked kinase (ILK) capable of directly modulating ILK expression or directly blocking ILK catalytic or binding activity in the manufacture of a medicament for the treatment of ocular neovascularisation.

The inhibitors are agents that interfere with the ILK signaling pathway, including integrin linked kinase (ILK) blocking agents; compounds that otherwise prevent the binding of natural ILK ligands to ILK; or compounds that prevent expression of, or signaling through, ILK. Such a treatment is used alone as single therapy or in combination with a second therapy as an adjunct to prevent, to reduce or to reverse the loss of visual acuity as well as loss of vision secondary to neovascularization of cornea, iris, retina or choroid.

In one aspect, the invention is directed to the use of an inhibitor of ink as defined above in the manufacture of a medicament to prevent, to reduce or to reverse ocular neovascularization in an eye of an animal having a neovascular lesion, comprising the steps of identifying said lesion in the eye of the animal, administering to the animal an amount of small molecule inhibitor of ILK sufficient to allow said compound to localize in said lesion.

In another aspect, the prevention, reduction of reversal is of a loss of visual acuity or vision loss in a patient having corneal, iris, retinal or choroidal neovascularization lesion associated with various eye diseases, comprising the steps of administering an amount of a ILK inhibitor sufficient to allow said inhibitor to localize in said lesion. In a preferred embodiment, an ILK inhibitor will be administered alone as single therapy. In another preferred embodiment, an ILK inhibitor will be administered at an appropriate time, before, concurrent or after, in relation to a second therapy including but not limited to photodynamic therapy such as Visudyne^{™} therapy, photocoagulation or transpupillary thermotherapy as an adjunct treatment for ocular neovascularization.

### BRIEF DESCRIPTION OF THE DRAWINGS

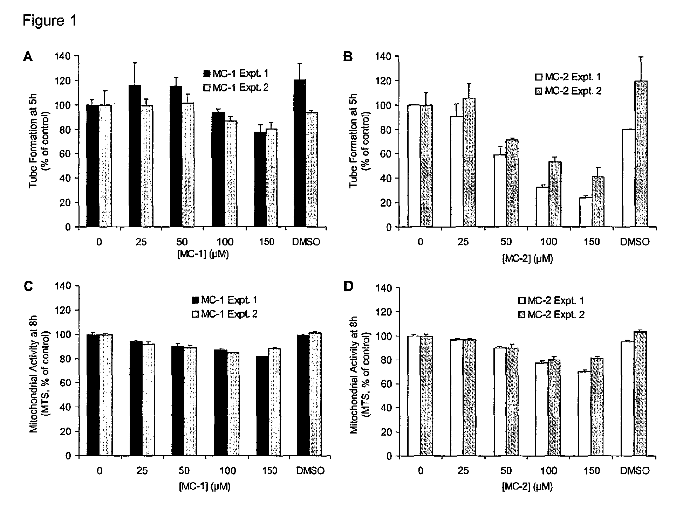

Figures 1A-1D show HUVEC human endothelial cells grown in a Matrigel support in the presence or absence of ILK inhibitor MC-1 (figures 1A and 1C), MC-2 (figures 1B and D) or solvent control (dimethyl sulfonamide, DMSO)

### DETAILED DESCRIPTION OF THE EMBODIMENT

As described herein, compounds that modulate the activity of integrin linked kinase (ILK) are administered systemically or locally to treat ophthalmic diseases with an underlining pathology that is characteristic of ocular neovascularization. Such a treatment is used alone as single therapy or in combination with a second therapy as an adjunct to prevent, to reduce or to reverse the loss of visual acuity as well as loss of vision secondary to neovascularization of cornea, iris, retina or choroid.

In one aspect, the invention is directed to the use of as inhibitor of ILK as defined above in the manufacture of a medicament to prevent, to reduce or to reverse ocular neovascularization in an eye of an animal having a neovascular lesion, comprising the steps of identifying said lesion in the eye of the animal, administering to the animal an amount of small molecule inhibitor of ILK sufficient to allow said compound to localize in said lesion. Methods utilizing local administration that provides for a prolonged localized concentration, which may utilize sustained release implants, viscous solutions, or other topical formulation, are of particular interest. An ILK inhibitor can be administered alone as single therapy, or in combination with a second therapy, for example at an appropriate time, before, concurrent or after, in relation to a second therapy including but not limited to phtotodynamic therapy, including but not limited to Visudyne^{™} therapy, photocoagulation or transpupillary thermotherapy as an adjunct treatment for ocular neovascularization.

Some examples of ocular disorders that may be treated by various embodiments of the present invention include, without limitation: retinal diseases (diabetic retinopathy, diabetic macular edema, chronic glaucoma, retinal detachment, sickle cell retinopathy, age related macular degeneration (AMD) due to subretinal neovascularization); rubeosis iritis; inflammatory diseases; chronic uveitis; neoplasms (retinoblastoma, pseudoglioma); Fuchs' heterochromic iridocyclitis; neovascular glaucoma; corneal neovascularization (inflammatory, transplantation, developmental hypoplasia of the iris); neovascularization resulting following a combined vitrectomy and lensectomy; vascular diseases (retinal ischemia, choroidal vascular insufficiency, choroidal thrombosis, carotid artery ischemia); neovascularization of the optic nerve; and neovascularization due to penetration of the eye or contusive ocular injury.

### ILK MODULATING AGENTS

ILK is a 59 kDa serine/threonine kinase that associates with the cytoplasmic tails of β1 and β3 integrins. The enzymatic activity for ILK is modulated by the interaction of cells with the extracellular matrix component fibronectin, integrin clustering and a number of growth factors. Because of its intimate association with a wide variety of signaling pathways that have been directly or indirectly implicated in various pathological processes, ILK may represent a therapeutic target for a variety clinical conditions including angiogenesis, cancer, inflammation and autoimmunity. The genetic sequence of human ILK is disclosed in U.S. Patent nos. 6,013,782; and 6,001,622.

Agents that block ILK activity provide a point of intervention in an important signaling pathway. Numerous agents are useful in reducing ILK activity, including agents that directly modulate ILK expression, e.g. expression vectors, anti-sense specific for ILK, ILK specific antibodies and analogs thereof, small organic molecules that block ILK catalytic activity, etc. For example, small molecule inhibitors of integrin linked kinase are described in U.S. Patent Nos. 6,214,813 and 6,436,915, 6,420,400 and in the respective Examples. Antisense inhibitors of ILK are described in U.S. Patent no. 6,177,273.

Agents that block ILK activity are used in the treatment of ocular disease relating to neovascularization. Numerous agents are useful in reducing ILK activity, including agents that directly modulate ILK expression, *e.g*. anti-sense specific for ILK, ILK specific antibodies and analogs thereof, small organic molecules that block ILK catalytic or binding activity, *etc*. For example, small molecule inhibitors of integrin linked kinase are described in U.S. Patent No. 6,214,813, in co-pending application U.S. Serial Number 09/747,563, and in the co-pending application entitled "Hydrazonopyrazole derivatives and their use as anti-proliferative agents". Antisense inhibitors of ILK are described in U.S. Patent no. 6,177,273.

Agents of interest for down-regulating ILK activity include direct blocking of [PtdIns(3,4,5)P₃] binding sites through competitive binding, steric hindrance, etc. Of particular interest are antibodies that bind to the PH domains, thereby blocking the site. Antibodies include fragments, *e.g.* F(Ab), F(Ab)', and other mimetics of the binding site. Such antibodies can be raised by immunization with the protein or the specific domain. Mimetics are identified by screening methods. Analogs of [Ptdlns(3,4,5)P₃] that compete for binding sites but do not result in activation of ILK are also of interest.

Because ILK activity is upregulated by the presence of the lipid [PtdIns(3,4,5)P₃], the activity of ILK can be manipulated by agents that block the binding of [PtdIns(3,4,5)P₃] to ILK. The amino acid sequence of ILK contains a sequence motif found in pieckstrin homology (PH) domains, which are involved in the binding of phosphatidylinositol phosphates. The inhibitors are administered *in vivo* or *in vitro* at a dose sufficient to provide for these concentrations in the target tissue.

Drug screening can be used to identify agents that modulate ILK function. One can identify ligands or substrates that inhibit the action of ILK. A wide variety of assays may be used for this purpose, including labeled *in vitro* protein-protein binding assays, electrophoretic mobility shift assays, immunoassays for protein binding, and the like. Knowledge of the 3-dimensional structure of ILK, derived from crystallization of purified recombinant ILK protein, leads to the rational design of small drugs that specifically inhibit ILK activity. These drugs may be directed at specific domains of ILK, e.g. the kinase catalytic domain, ankyrin repeat domains, pleckstrin homology domains, etc. Among the agents of interest for drug screening are those that interfere with the binding of cytoplasmic integrin tails to ILK; the kinase activity of ILK; or binding of [Ptdlns(3,4,5)P₃] to the PH domains of ILK.

The term "agent" as used herein describes any molecule, *e.g.* protein or pharmaceutical, with the capability of altering the physiological function of ILK. Candidate agents encompass numerous chemical classes, though typically they are organic molecules, preferably small organic compounds having a molecular weight of more than 50 and less than about 2,500 daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof.

Assays of interest may detect agents that block ILK function, such as integrin binding, kinase activity, down regulation of E-cadherin, up regulation of LEF-1, binding properties, *etc*. For example, an expression construct comprising a ILK gene may be introduced into a cell line under conditions that allow expression. The level of ILK activity is determined by a functional assay, as previously described. In one screening assay, candidate agents are added, and the formation of fibronectin matrix is detected. In another assay, the ability of candidate agents to enhance ILK function is determined.

### METHODS OF TREATMENT

The subject methods are used for prophylactic or therapeutic purposes to treat ocular diseases to prevent, reduce or reverse the loss of visual acuity as well as loss of vision secondary to neovascularizatlon of cornea, iris, retina or choroid. Use used herein, the term "treating" is used to refer to both prevention of disease, and treatment of pre-existing conditions. While treatment during early stages is desirable, the adverse symptoms of the disease may be at least partially alleviated by treatment during later stages.

In practicing the use of the present invention, a therapeutically effective amount of an ILK inhibitor is administered to a subject afflicted with a disease or disorder related to neovascularization, or to a tissue that has been neovascularized. The inhibitor may be administered in accordance with the method of the invention either alone of in combination with other known therapies for neovascularization. When co-administered with one or more other therapies, the inhibitor may be administered either simultaneously with the other treatment(s), or sequentially. If' administered sequentially, the attending physician will decide on the appropriate sequence of administration, which may be before or after a second therapy.

Secondary therapies of interest include photodynamic therapy, including, but not limited to verteporfin (VISUDYNE^{™}) therapy, see, for example Madreperla (2001) Arch Ophthalmol. **119**(11):1606-1610; Harding (2001) Eye **15**(Pt 3):407-12; Sharma (2001) Can Fam Physician. **47**:955, 963, U.S. 55,756,541 "Vision through photodynamic therapy of the eye", Photocoagulation or transpupillary thermotherapy, see, for example Rogers *et al*. (2001) Curr Opin Ophthalmol. **12**(3):212-5; Ardjomand *et al*. (2001) Ophthalmologica. **215**(3):241-4; Mainster *et al*. (2000) Ophthalmic Surg Lasers, **31**(5):359-73. Other therapies include, without limitation, those set forth in U.S. 6,297,228, "Use of angiostatic steroids in photodynamic therapy", U.S. 6,271,233 "Method for treating ocular neovascular diseases"; U.S. 6,248,734 "Use of photodynamic therapy for prevention of secondary cataracts"; U.S. RE37,180 "Photochemotherapeutical obstruction of newly-formed blood vessels"; U.S. 6,225,303 and 5,798,349 "Use of green porphyrins to treat neovasculature in the eye"; U.S. 6,217,895 "Method for treating and/or preventing retinal diseases with sustained release corticosteroids"; U.S. 6,214,819 "Method for treating ocular neovascular diseases", and the like.

When photodynamic therapy is used in conjunction with ILK inhibitors in the treatment of ocular neovascular diseases, a wide range of photosensitizers may be used, including, but not limited to, hematoporphyrin derivatives, pheophorbides, chlorins, bacteriochlorins, phthalocyanines, purpurins, merocyanines, texaphryns and green porphyrins, as well as protoporphyrin precursors such as aminolevulinic acid (ALA) and derivatives thereof. Each photosensitizer can be activated with light containing a wavelength absorbed by the photosensitizer. The photosensitizers may be administered locally or systemically, preferably by injection. The dose of ILK inhibitor in the pharmaceutical composition of the present invention will depend upon the nature and severity of the condition being treated, and on the nature of prior treatments which the patent has undergone. Ultimately, the attending physician will decide the dose with which to treat each individual patient. Initially, the attending physician may administer low doses and observe the patient's response. Larger doses may be administered until the optimal therapeutic effect is obtained for the patient, and at that point the dosage is not increased further.

Some diseases lend themselves to acute treatment while others require to longer term therapy. Proliferative retinopathy can reach a threshold in a matter of days as seen in ROP, some cases of diabetic retinopathy, and neovascular glaucoma. Premature infants are at risk for neovascularization around what would be 35 weeks gestation, a few weeks after birth, and will remain at risk for a short period of time until the retina becomes vascularized.

Suitable animal models exist for determination of appropriate dosage, although the efficacy of a therapeutic effect for different mammals varies widely, for example doses typically are 20, 30 or even 40 times smaller (per unit body weight) in man than in the rat. Similarly the mode of administration can have a large effect on dosage. A murine model of oxygen-induced retinal neovascularization has been established which occurs in 100% of treated animals and is quantifiable (Smith *et al*. (1994) Invest. Ophthalmol. Vis. Sci. **35:**101-111). Bioactivity can be determined by methods including the Miles vessel permeability assay (Miles and Miles (1952) J. Physiol. (Lond.) 118:228), which measures vessel permeability, and endothelial cell mitogenicity, which measures cell growth. Other suitable models are set forth in the Examples.

The compounds of this invention can be incorporated into a variety of formulations for therapeutic administration. Administration of an ILK inhibitor may be by delivery using any appropriate means including, but not limited to, systemic, local, or even direct application to the target tissue. Local delivery of an ILK inhibitor provides a high local concentration while reducing the likelihood of non-specific anti-angiogenic or other undesirable side effects that may follow systemic administration of an ILK inhibitor.

For local application, a range of about 0.05 to 0.2 or about 0.5 to 2.0 mg/ml of an ILK inhibitor in an appropriate formulation is administrated either intra-ocularly (intra-vitreous, subretinal, intra-anterior chamber, intra-scleral), peri-ocularly (topically onto the cornea, subconjunctival, subtenon, transcleral). For systemic application, a range of 0.05 to 100 mg /kg body weight, preferably less than about 10 mg/kg is administered.

For intra- or peri-ocular administration, an ILK inhibitor in an injectable formulation can be administered by either an intra-ocular injection at above-described concentrations and at a frequency of once every 2-6 months or by an intra-ocular implantation of a device or a specific formulation of an ILK inhibitor allowing sustained release of the ILK inhibitor over a period of time. For corneal application, an ILK inhibitor in an appropriate formulation can be applied topically onto the cornea at a frequency of once very 4-6 hours. For systemic application, an ILK inhibitor in appropriate formulation can be administered orally 1-3 times a day.

The compounds of the present invention are formulated into pharmaceutical compositions by combination with appropriate, pharmaceutically acceptable carriers or diluents, and may be formulated into preparations in solid, semi-solid, liquid or gaseous forms, such as tablets, capsules, powders, granules, ointments, solutions, suppositories, injections, inhalants, gels, microspheres, and aerosols. As such, administration of the compounds can be achieved in various ways, including oral, buccal, rectal, parenteral, intraperitoneal, intradermal, transdermal, intracheal, *etc*., administration. The ILK may be systemic after administration or may be localized by the use of an implant that acts to retain the active dose at the site of implantation.

The compounds of the present invention can be administered alone, in combination with each other, or they can be used in combination with other known compounds and therapies. In pharmaceutical dosage forms, the compounds may be administered in the form of their pharmaceutically acceptable salts, or they may also be used alone or in appropriate association, as well as in combination with other pharmaceutically active compounds.

For oral preparations, the compounds can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

The compounds can be formulated into preparations for injections by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

Implants for sustained release formulations are well-known in the art. Implants are formulated as microspheres, slabs, *etc*. with biodegradable or non-biodegradable polymers. For example, polymers of lactic acid and/or glycolic acid form an erodible polymer that is well-tolerated by the host. The implant is placed in proximity to the site of infection, so that the local concentration of active agent is increased relative to the rest of the body.

The term "unit dosage form," as used herein, refers to physically discrete units suitable as unitary dosages for human and animal subjects, each unit containing a predetermined quantity of compounds of the present invention calculated in an amount sufficient to produce the desired effect in association with a pharmaceutically acceptable diluent, carrier or vehicle. The specifications for the novel unit dosage forms of the present invention depend on the particular compound employed and the effect to be achieved, and the pharmacodynamics associated with each compound in the host.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

Those of skill will readily appreciate that dose levels can vary as a function of the specific compound, the severity of the symptoms and the susceptibility of the subject to side effects. Some of the specific compounds are more potent than others. Preferred dosages for a given compound are readily determinable by those of skill in the art by a variety of means. A preferred means is to measure the physiological potency of a given compound.

It is to be understood that this invention is not limited to the particular methodology, protocols, cell lines, animal species or genera, constructs, and reagents described, as such may vary. It is also to be understood that the terminology used herein is for the purpose of describing particular embodiments only, and is not intended to limit the scope of the present invention which scope will be determined by the language in the claims.

It must be noted that as used herein and in the appended claims, the singular forms "a", "and", and "the" include plural referents unless the context clearly dictates otherwise. Thus, for example, reference to "a mouse" includes a plurality of such mice and reference to "the cytokine" includes reference to one or more cytokines and equivalents thereof known to those skilled in the art, and so forth.

Unless defined otherwise, all technical and scientific terms used herein have the same meaning as commonly understood to one of ordinary skill in the art to which this invention belongs- Although any methods, devices and materials similar or equivalent to those described herein can be used in the practice or testing of the invention, the preferred methods, devices and materials are now described.

The following examples are put forth so as to provide those of ordinary skill in the art with a complete disclosure and description of how to make and use the subject invention, and are not intended to limit the scope of what is regarded as the invention. Efforts have been made to ensure accuracy with respect to the numbers used (*e.g.* amounts, temperature, concentrations, *etc.*) but some experimental errors and deviations should be allowed for. Unless otherwise indidated, parts are parts by weight, molecular weight is average molecular weight, temperature is in degrees centigrade; and pressure is at or near atmospheric.

### EXPERIMENTAL

### EXAMPLE 1

### Treatment of AMD using an ILK inhibitor as an adjunct to Visudyne^{™} therapy

Therapeutic effect of an ILK inhibitor in AMD is evaluated using visual acuity as the primary clinical outcome. Patients with subforveal CNV lesions caused by AMD are examined for the presence of lesions that meet the inclusion criteria. The inclusion criteria are defined as the presence of lesions measuring 5400 µm or less in greatest linear dimension with evidence of classic CNV and best-corrected visual acuity of approximately 20/40 to 20/200 based on fluorescein angiographic and visual acuity examination. Those determined as qualified for the treatment of AMD are randomly assigned to 4 groups. Group A, B, and C are treated with standard Visudyne^{™} therapy with an adjunct therapy using an ILK inhibitor. Patients of Group D are treated with standard Visudyne^{™} therapy in combination with a placebo of the ILK inhibitor.

For standard Visudyne^{™} therapy, patients are administered 30 ml of Visudyne^{™} (0.15 mg per kilogram of body weight). The active ingredient in Visudyne^{™} is verteporfin, also known as BPD-MA (U.S. 5,095,030). The administration is by intravenous infusion over a period of 10 minutes. Fifteen minutes after the end of the infusion, the laser light is applied for 83 seconds to the CNV lesion through a fundus contact lens of known magnification to result in a light exposure of 50 J/cm². A circular spot of approximately 6000 microns encompassing the area of the lesion is exposed to the laser light.

For the adjunct therapy, patients of groups A, B, and C receive a daily oral administration of an ILK inhibitor described in U.S. Patent No. 6,214,813, in co-pending application U.S. Serial Number 09/747,563, or in the co-pending application entitled "Hydrazonopyrazole derivatives and their use as anti-proliferative agents" at the dose of 5, 10, 20 mg per kilogram body weight, respectively. The adjunct treatment commences three days after the patient receives the standard Visudyne^{™} therapy and continues for a period of one month.

As follow-up, patients are examined every three months. At each regularly scheduled follow-up visit, best-corrected visual acuity measurement, contrast threshold measurement, ophthalmoscopic examination, stereoscopic fundus photography, and fluorescein angiography are performed.

Alternatively, patients having subforveal CNV lesions caused by AMD receive a daily oral dose of an ILK inhibitor as described above as a stand alone therapy, but do not receive Visudyne^{™} therapy.

### Example 2

### Treatment of diabetic retinopathy using an ILK inhibitor

Therapeutic effect of an ILK inhibitor in proliferative diabetic retinopathy is evaluated using visual acuity as the primary clinical outcome. Patients with proliferative diabetic retinopathy and visual acuity of 20/100 or better in each eye are included in the clinical evaluation. Patients are randomly assigned to 3 treatment groups and 1 placebo group. Group A, B, and C are treated with daily oral administration of an ILK inhibitor as described in Example 1 at the dose of 5, 10, 20 mg per kilogram body weight. Patients of Group D receive placebo. The treatment continues for a period of 24 months.

As follow-up, patents are examined every 4 months. At each regularly scheduled follow-up visit, best-corrected visual acuity measurement, contrast threshold measurement, indirect ophthalmoscopic examination, stereoscopic fundus photography, fluorescein angiography, and slit-lamp examination using 78- or 90-diopter lens are performed.

### Example 3

### Evaluation of ILK expression in ocular vascular tissue

This example documents the discovery that ILK is a therapeutic target for diseases with underling pathology of ocular neovascularization.

Post mortem baboon eye samples were subjected to immunohistological analysis for the expression of ILK in ocular vasculature. Freshly obtained tissues were snap-frozen by immersing into a Dewar of liquid nitrogen. Cross sections of 5-10 microns were prepared and fixed in cold acetone (-20 C). Immunohistology was performed using a rabbit anti-ILK antibody (Upstate Biotechnology Institute, NY. Cat.# 06-550) and Zymed Histostatin^{™} Plus kit (Zymed, Cat.#85-9743).

Abundant expression of ILK was detected in choroidal and retinal endothelium in post mortem baboon eye samples. Under similar condition, no significant level of ILK expression was detected in retinal pigmented epithelial cells. In addition, no significant expression of ILK in neurons and photoreceptors was observed.

### Example 4

### ILK inhibitor MC-2 (KP-31861 as found in co-pending patent application serial no. 10/077,238) inhibits angiogenesis in an in vitro model of angiogenesis

The following model was used to evaluate anti-angiogenic activity of ILK inhibitors. This model provides a convenient short term quantifiable *in vitro* measurement of the anti-angiogenic activity of ILK inhibitor. HUVEC human endothelial cells were grown in a Matrigel support (VWR Catalog No. CACB 354234) in the presence or absence of ILK inhibitor MC-1 (Figure 1, panels A and C), MC-2 (figure X, panels B and D) or solvent control (dimethyl sulfonamide, DMSO). Tube formation was quantified by a computer-assisted image analysis method using Image Pro Plus (Media Cybernetics, ML) measuring total tube length as captured by the microscope in each well at 5 h after application of the ILK inhibitor. Cell viability was determined by measuring mitochondrial dehydrogenase activity in the culture supernatant at 8 h using the MTS (3-(4,5-dimethylthiozol-2-yl)-5-(3-carboxymethoxyphenyl-2-(4-sulfophenyl)-2H-tetrazolium) proliferation assay (Promega Corporation, FL, USA., Pat. No. TB169). The MTS reagent was added to the culture at 5 h after application of the ILK inhibitor. The cells were incubated at 37°C for additional 3 h and culture supernatant was collected and absorbance at 490 nm was measured with an ELISA plate reader.

An inhibitory effect on endothelial tube formation was detected with MC-2. About 35, 60 or 70% inhibition of tube formation was observed at 5 h after the application of 50, 100 or 150 µM of MC-2, respectively. However, cell viability measured by metabolic activity of the cells was not significantly affected by ILK inhibitor MC-2 based on all MTS measurement of supernatant collected at 8 h from the same culture. For MC-2, the metabolic activity was about 90, 80, and 75% of the control levels at these concentrations.

### Example 5

### Treatment of corneal neovascularization with an ILK inhibitor using a mouse model

The following model provides a quantifiable *in vivo* assay that can be used to evaluate anti-angiogenic activity of an ILK inhibitor. Corneal neovascularization is induced by a procedure known as silver nitrate cauterization. The procedure involves topical applications of silver nitrate onto the cornea by gently touching conjunctiva/limbus for one second followed by touching the central cornea of an anesthetized mouse for 8 seconds with a silver nitrate applicator (Graham-Field, NY, Item # 1590, 75% silver nitrate, 25% potassium nitrate). Immediately after, the eye is rinsed with 10 ml of saline followed by topical application of Gentak Ophthalmic Ointment (0.3%, Gentamicin sulfate) on the eye to prevent bacterial infections.

Corneal neovascularization is recorded and evaluated by examining and photographing the cornea daily using a stereo dissecting microscope connected to a color video camera and a computer. Angiogenesis is evaluated based on new blood vessel growth within previous avascular cornea using a scoring system (score of 0-4) that rates from no neovascularization to very severe neovascularization in cornea. In addition, upon completion of the experiment (day 5-7), corneal neovascularization is quantified using computer-assisted image analysis (Image Pro Plus, Media Cybernetics, ML) of dye-stained blood vessels in post mortem whole corneal mounts. Corneal vasculature is stained by IV injection of high molecular weight FITC-dextran into anesthetized mice before euthanasia.

Animals receive daily intra-peritoneal administration of an ILK inhibitor at the dose of 5, 25 or 50 mg/kg commencing on day-2 after the silver nitrate cauterization procedure until 24 h before the ending of the experiment. Corneal neovascularization of ILK inhibitor-treated animals is compared with that of vehicle-treated animals.

### Example 6

### Treatment of choroidal neovascularization with an ILK inhibitor using a monkey model of CNV

The following model provides an *in vivo* assay that can be used to evaluate therapeutic potential of ILK inhibitors for the treatment of CNV. CNV is induced by argon green laser burns that are placed in the maculae of cynomolgus monkeys using a modification of Ryan's model, as described in U.S. 5,798,349. The laser burn with size of 50 µm in diameter is induced by exposure to 350-450 mW laser light at 514 nm for 0.1 second using an argon laser (Coherent Argon Dye Laser #920, Coherent Medical Laser, Polo Alto, CA).

CNV is monitored by weekly examination with fundus photography and fluorescein angiography. At the termination of the experiment (2-3 months after the induction of CNV), eyes are enucleated under deep anesthesia and fixed in modified Kanovsky fixative. Bisection is performed 20 min after fixation. Tissues are then embedded and sections are generated for histological and immunohistological analysis using antibodies against vasculature-specific markers including CD-31 and VE-Cadherin. The extent of neovascularization is quantified using a computer-assisted image analysis system with Image Pro Plus (Media Cybernetics, ML).

Animals receive daily oral administration of an ILK inhibitor at the dose of 10, 50 or 100 mg/kg for commencing after the onset of CNV (2-3 weeks after the laser treatment). As control, a group of monkeys receive daily oral treatment with vehicle only. CNV in ILK inhibitor-treated animals is compared with that of vehicle-treated animals for angiographic and immunohistological evidence of CNV.

### Example 7

### Treatment of retinal neovascularization with an ILK inhibitor using a mouse model of ischmia-induced retinopathy

The following model provides an *in vivo* assay that can be used to evaluate therapeutic potential of ILK inhibitors for the treatment of retinopathy. This is a mouse model of retinopathy of prematurity. Retinopathy in mice is induced by using dams and neonatal mice. Mice are exposed with their nursing dams to 75% oxygen/25% nitrogen from postnatal day 7 to day 12, then put back to room air.

At day 17, all pups are weighed, euthanised, and perfused with 1 ml fixative (4% paraformaldhyde/8% sucrose/sodium phosphate buffer, pH 7.2) through the left ventricle of heart. Eyes are enucleated and placed in fixative. The fixed tissues are paraffin-embedded and 4-µm sections are cut. Immunohistology procedure is performed to evaluate extent of retinal neovascularization using antibodies against endothelium-specific markers including CD-31 and VE-cadherin. The vascular specific staining is quantified using the computer-assisted image analysis method (Image Pro Plus, Media Cybernetics, ML).

The ILK inhibitor at the dose of 5, 25 or 50 mg/kg is administered daily through intra-peritoneal injection from day 12 through day 16. The control group receives daily injection of vehicle. The inhibitory effect of the ILK inhibitor on retinal neovascularization is determined by comparing the extent of vascular staining in mice treated with the ILK inhibitor and those treated with vehicle only.

## Claims

1. The use of an inhibitor of integrin linked kinase (ILK) capable of directly modulating ILK expression or directly blocking ILK catalytic or binding activity in the manufacture of a medicament for the treatment of ocular neovascularisation.

2. The use of claim 1 wherein said treatment reduces or reverses the loss of visual acuity secondary to neovascularisation or cornea, iris, retina or choroid.

3. The use of claim 1 or 2 further comprising administering a second therapy for neovascularisation.

4. The use of claim 3 wherein the second therapy is selected from the group consisting of Visudyne® therapy, photocoagulation and transpupillary thermotherapy.

5. The use of claims 1 to 3 wherein said ocular neovascularisation is selected from the group consisting of diabetic retinopathy, chronic glaucoma, retinal detachment, sickle cell retinopathy, age related macular degeneration (AMD) due to subretinal neovascularisation, rubeosis iritis, inflammatory diseases, chronic uveitis, neoplasms, Fuchs' heterochromic iridocyclitis, neovascular glaucoma, corneal neovascularisation, neovascularisation resulting following a combined vitrectomy and lensectomy, retinal ischemia, choroidal vascular insufficiency, choroidal thrombosis, carotid artery ischemia, neovascularisation of the optic nerve and neovascularisation due to penetration of the eye or contusive ocular therapy.

6. The use of any one of claims 1 to 5 wherein said ILK inhibitor is administered systemically.

7. The use of any one of claims 1 to 5 wherein said ILK inhibitor is administered locally,

8. The use of claim 7 wherein said local administration utilises sustained release implants.

9. The use of claim 7 wherein said local administration utilises a topical formulation.

10. The use of any one of claims 1 to 6 wherein said ILK inhibitor is administered intra-ocularly.

11. The use of any one of claims 1 to 6 wherein said ILK inhibitor is administered peri-ocularly.

12. The use of claim 9 wherein said ILK inhibitor is administered topically onto the cornea.

13. The use of claim 10 wherein said ILK inhibitor is administered by intra-ocular injection.

14. The use of claim 10 wherein said ILK inhibitor is administered by intra-ocular implantation.

15. The use of any one of claims 1 to 14 wherein said ILK inhibitor is anti-sense specific for ILK.

16. The use of any one of claims 1 to 14 wherein said ILK inhibitor is an ILK specific antibody and analog thereof.

17. The use of any one of claims 1 to 14 wherein said ILK inhibitor is a small organic molecule that blocks ILK catalytic or binding activity.

## Patentansprüche

1. Verwendung eines Inhibitors von integringekoppelter Kinase (ILK), der zur direkten Modulation der ILK-Expression oder direkten Blockade der ILK-Katalyse- oder -Bindungsaktivität in der Lage ist, zur Herstellung eines Medikaments zur Behandlung von okularer Neovaskularisation.

2. Verwendung nach Anspruch 1, worin die Behandlung den Verlust von Sehschärfe sekundär zu einer Neovaskularisation der Cornea, Iris, Retina oder Chorioidea verringert oder umkehrt.

3. Verwendung nach Anspruch 1 oder 2, weiters die Verabreichung einer zweiten Neovaskularisationstherapie umfassend.

4. Verwendung nach Anspruch 3, worin die zweite Therapie aus der aus einer Visudyne®-Therapie, Photokoagulation und transpupillarer Thermotherapie bestehenden Gruppe ausgewählt ist.

5. Verwendung nach einem der Ansprüche 1 bis 3, worin die okulare Neovaskularisation aus der aus Retinopathia diabetica, Weitwinkelglaukom, Netzhautablösung, Sichelzellenretinopathie, altersbedingter Makuladegeneration (AMD) aufgrund von subretinaler Neovaskularisation, Rubeosis iridis, Entzündungserkrankungen, chronischer Uveitis, Neoplasmen, heterochromer Fuchs-Zyklitis, neovaskulärem Glaukom, Corneaneovaskularisation, Neovaskularisation nach einer kombinierten Vitrektomie-Lentektomie, retinaler Ischämie, vaskulärer Insuffizienz in der Chorioidea, Chorioideathrombose, Karotisischämie, Neovaskularisation des Sehnervs und Neovaskularisation aufgrund einer Penetration des Auges oder der Therapie einer okularen Quetschung bestehenden Gruppe ausgewählt ist.

6. Verwendung nach einem der Ansprüche 1 bis 5, worin der ILK-Inhibitor systemisch verabreicht wird.

7. Verwendung nach einem der Ansprüche 1 bis 5, worin der ILK-Inhibitor lokal verabreicht wird.

8. Verwendung nach Anspruch 7, worin die lokale Verabreichung mittels Retardpräparatimplantaten erfolgt.

9. Verwendung nach Anspruch 7, worin die lokale Verabreichung mittels einer topischen Formulierung erfolgt.

10. Verwendung nach einem der Ansprüche 1 bis 6, worin der ILK-Inhibitor intraokular verabreicht wird.

11. Verwendung nach einem der Ansprüche 1 bis 6, worin der ILK-Inhibitor periokular verabreicht wird.

12. Verwendung nach Anspruch 9, worin der ILK-Inhibitor topisch auf die Cornea verabreicht wird.

13. Verwendung nach Anspruch 10, worin der ILK-Inhibitor durch intraokulare Injektion verabreicht wird.

14. Verwendung nach Anspruch 10, worin der ILK-Inhibitor durch intraokulare Implantation verabreicht wird.

15. Verwendung nach einem der Ansprüche 1 bis 14, worin der ILK-Inhibitor antisensespezifisch für ILK ist.

16. Verwendung nach einem der Ansprüche 1 bis 14, worin der ILK-Inhibitor ein ILK-spezifischer Antikörper und ein Analogon davon ist.

17. Verwendung nach einem der Ansprüche 1 bis 14, worin der ILK-Inhibitor ein kleines organisches Molekül ist, das die ILK-Katalyse- oder -Bindungsaktivität blockiert.

## Revendications

1. Utilisation d'un inhibiteur de la kinase liée aux intégrines (ILK, *integrin linked kinase*) capable de moduler directement l'expression d'ILK ou de bloquer directement l'activité catalytique ou l'activité de liaison de l'ILK dans la fabrication d'un médicament destiné au traitement de la néovascularisation oculaire.

2. Utilisation selon la revendication 1, dans laquelle ledit traitement réduit ou inverse la perte d'acuité visuelle consécutive à la néovascularisation de la cornée, de l'iris, de la rétine ou de la choroïde.

3. Utilisation selon la revendication 1 ou la revendication 2, comprenant en plus l'administration d'une deuxième thérapie anti-néovascularisation.

4. Utilisation selon la revendication 3 , dans laquelle la deuxième thérapie est choisie dans le groupe constitué par la thérapie Visudyne®, la photocoagulation et la thermothérapie transpupillaire.

5. Utilisation selon les revendications 1 à 3, dans laquelle ladite néovascularisation oculaire est choisie dans le groupe constitué par la rétinopathie diabétique, le glaucome chronique, le décollement de la rétine, la rétinopathie à cellules falciformes, la dégénérescence maculaire liée à l'âge (DMLA) due à la néovascularisation sous-rétinienne, l'iritis avec rougeur, des maladies inflammatoires, l'uvéite chronique, des néoplasmes, l'iridocyclite hétérochromique de Fuchs, le glaucome néovasculaire, la néovascularisation cornéenne, la néovascularisation consécutive à une vitrec tomie et une phakectomie combinées, l'ischémie rétinienne, l'insuffisance vasculaire choroïdienne, la thrombose choroïdienne, l'ischémie des artères carotides, la néovascularisation du nerf optique et la néovascularisation due à une pénétration de l'oeil ou à une thérapie oculaire contusive.

6. Utilisation selon l'une quelconque des revendications 1 à 5, dans laquelle ledit inhibiteur de l'ILK est administré par voie systémique.

7. Utilisation selon l'une quelconque des revendications 1 à 5, dans laquelle ledit inhibiteur de l'ILK est administré localement.

8. Utilisation selon la revendication 7, dans laquelle ladite administration locale utilise de s implants à libération entretenue.

9. Utilisation selon la revendication 7, dans laquelle ladite administration locale utilise une formulation à usage local.

10. Utilisation selon l'une quelconque des revendications 1 à 6, dans laquelle ledit inhibiteur de l'ILK est administré par voie intraoculaire.

11. Utilisation selon l'une quelconque des revendications 1 à 6, dans laquelle ledit inhibiteur de l'ILK est administré par voie périoculaire.

12. Utilisation selon la revendication 9, dans laquelle ledit inhibiteur de l'ILK est administré localement sur la cornée.

13. Utilisation selon la revendication 10, dans laquelle ledit inhibiteur de l'ILK est administré par injection intraoculaire.

14. Utilisation selon la revendication 10 dans, laquelle ledit inhibiteur de l'ILK est administré par implantation intraoculaire.

15. Utilisation selon l'une quelconque des revendications 1 à 14, dans laquelle ledit inhibiteur de l'ILK est spécifique antisens de l'ILK.

16. Utilisation selon l'une quelconque des revendications 1 à 14, dans laquelle ledit inhibiteur de l'ILK est un anticorps et un analogue d'anticorps spécifique à l'ILK.

17. Utilisation selon l'une quelconque des revendications 1 à 14, dans laquelle ledit inhibiteur de l'ILK est une petite molécule organique qui blo que l'activité cata lytique ou l'activité de liaison de l'ILK.
